# EUROPEAN PATENT SPECIFICATION

(11) **EP 2 606 741 B1**
(45) Date of publication and mention of the grant of the patent: **16.04.2014**
(21) Application number: 11195701.5
(22) Date of filing: 23.12.2011
(51) Int. Cl.: A23J 1/06, A23K 1/04, A23K 1/18, A61K 35/16

(54) **Glycoprotein enriched composition as a food and feed additive and/or as a therapeutic agent**
Glycoprotein-angereicherte Zusammensetzungen als Lebensmittel- und Futterzusatz und/oder als therapeutischer Wirkstoff
Composition enrichie à la Glycoprotéine en tant qu'aliment et additif alimentaire et/ou agent thérapeutique

(43) Date of publication of application: 26.06.2013
(73) Proprietor: FOODIP SARL, 1258 Luxembourg (LU)
(72) Inventor: De Buyser, Dirk, 8210 Zedelgem (BE); Heens, Benoit, 8950 Heuvelland (BE)
(74) Representative: De Clercq, Ann G. Y.

(56) References cited:
- WO-A1-00/56166
- CN-A- 1 923 028
- US-A- 4 096 244
- US-A- 5 372 811
- US-A1- 2003 190 314
- A. OWUSU-ASIEDU, S. K. BAIDOO, C. M. NYACHOTI AND R. R. MARQUARDT: "Response of early-weaned pigs to spray-dried porcine or animal plasma-based diets supplemented with egg-yolk antibodies against enterotoxigenic Escherichia coli", J ANIM SCI, [Online] vol. 80, 2002, pages 2895-2903, XP002677483, Retrieved from the Internet: URL:http://jas.fass.org/content/80/11/2895 .full.pdf+html> [retrieved on 2012-06-11]

## Description

### FIELD OF THE INVENTION

The present invention relates to glycoprotein enriched compositions and their use in the treatment and/or prevention of diseases, more particular gastro-intestinal diseases. The present invention further relates to the use of a glycoprotein enriched composition as a food or feed additive.

### BACKGROUND

One of the problems for young children and young animals such as piglets, calves, puppies, kittens, etc., is the occurrence of diarrhoea, especially when weaning has stopped.

For instance, after weaning, enteric diseases of piglet are one of the most common causes of morbidity and mortality. Enteric diseases in factory farming and livestock cause huge economic losses through reduced growth performances. Reducing post-weaning diarrhoea is one of the main challenges for the pig industry. In commercial practice, the use of different additives has been recommended as a way to help the piglet during this phase. The integrity of the intestinal barrier is fundamental to the proper functioning of the epithelial cells and to prevent the entry of pathogenic bacteria. Several studies confirmed that intestinal permeability increases with diarrhoea.

The diarrhoea of young animals and young children is mainly caused by pathogenic organisms. For causing diarrhoea pathogenic organisms (e.g. bacteria like Entero Toxigenic Escherichia Coli (ETEC), viruses (e.g. parvovirus) or parasites (e.g. Giardia) should adhere to the intestine receptors of the intestine. The use of blood plasma as feed additive for the prevention of diarrhoea by *E. coli* has been vastly described in literature as well as the effects of spray-dried animal plasma and immunoglobulins on performance of early weaned pigs and calves. The action of feed plasma is typically explained by the presence of immunoglobulins, acting as antibody-antigen on pathogenic organisms. Immunoglobulins comprise typically light and heavy chain subunits bound by SS-bridges. These glycoproteins have a high molecular weight in non-reduced SDS-PAGE but split under reduction to low molecular weight moieties in a reduced SDS-PAGE. SDS-PAGE analysis under non-reduced and under reduced conditions can make a clear distinction between the immunoglobulins and the glycoproteins consisting of non intermolecular SS-bound glycoprotein-subunits.

PCT application WO 03/030918 relates to a pharmaceutical product or food supplement which contains a preparation from blood plasma as the active component in which the preparation contains a larger amount of complement system and/or antibodies (immunoglobulins) than normally present. The complement system (CS) is a group of glycoproteins which is capable of interaction under specific conditions, leading to an enhanced immune response. The CS comprises proteins which are involved in the activation of immune cells, thereby acting directly on the immune system. The compositions according to WO 03/030918 require the removal of fibrinogen in order to avoid interference when liquid products are prepared from the composition, thereby requiring additional preparation steps in order to remove fibrinogen. Also the compositions according to WO 03/030918 are particularly heat sensitive, thereby excluding any type of heat treatment, something not evident when providing food supplements or pharmaceutical products where sterility is a requirement.

Anti-microbial drugs are used in animal feed not only for therapeutic use. However, misuse and over-use of antibiotics has favoured the growth of resistant organisms, leading to problems with disease control. Thus, there is a need for agents which have similar effects as antibiotics but do not entail the risk of resistance.

### SUMMARY OF THE INVENTION

The present invention is based on the observation that glycoprotein enriched compositions can be advantageously used to reduce the incidence and severity of enteric diseases.

In a first aspect of the invention relates to a glycoprotein enriched composition comprising an at least 10% higher concentration of glycoproteins compared to the natural glycoprotein source wherein the enriched glycoprotein fraction consist essentially of non intermolecular SS-bound glycoprotein-subunits as measured by non-reduced and reduced SDS-PAGE. More particularly said glycoprotein enriched composition is prepared from human blood plasma, porcine blood plasma, bovine blood plasma, equine blood plasma, ovine blood plasma or avian blood plasma.

In a particular embodiment the present invention relates to a glycoprotein enriched composition according to the present invention wherein said glycoprotein enriched composition is prepared from eggs, whey or other glycoprotein containing natural sources. More particularly said glycoprotein enriched composition is further characterized by comprising an at least 10% lower albumin concentration compared to the natural glycoprotein source.

In a further particular embodiment said glycoprotein enriched composition according to the present invention is prepared from non-immunized blood plasma.

According to another embodiment the present invention relates to a food or feed supplement in solid or liquid form, characterized in that said food or feed supplement comprises a glycoprotein enriched composition according to the present invention. More particularly said food or feed supplement further comprises an animal feed selected from the group consisting of one or more of corn, sorghum, meat, barley, wheat, soybean meal, peanut, canola, whey, milk products, blood meal, bone meal, fish meal, fats and oils, amino acids, vitamins and minerals, to form a ration.

According to yet another embodiment the present invention relates to a glycoprotein enriched composition or a food or feed supplement according to the present invention for use as a medicament, more particularly for use in the treatment and/or prevention of foodborne diseases of young mammals, more particularly for use in the treatment and/or prevention of gastro-intestinal disease, more particularly for use in the treatment and prevention of enteric disease and more particularly for use in the treatment and prevention of diarrhoea, and more preferably post weaning diarrhoea.

According to yet another embodiment the present invention relates to the use of a glycoprotein enriched composition or a food or feed supplement according to the present invention as a dietary supplement.

### BRIEF DESCRIPTION OF THE FIGURES

The present invention will be illustrated by the following figures which are intended for illustrative purposes only and the invention should in no way be considered to be limited thereto.
**Figure 1** represents a SDS-PAGE gel separation under reducing circumstances.
**Figure 2** represents a SDS-PAGE gel separation under non-reducing circumstances.
**Figure 3** represents a SDS-PAGE gel separation under reducing wherein the non non-immunoglobulin glycoprotein fraction is increased.
**Figure 4** represents a SDS-PAGE gel separation under reducing wherein the non non-immunoglobulin glycoprotein fraction is increased.
**Figure 5** is a graph illustrating the effectiveness of the glycoprotein enriched compositions according to the present invention.

### DETAILED DESCRIPTION OF THE INVENTION

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. Nevertheless, where the term comprising is used to refer to the presence of a number of elements or steps this is meant to also include embodiments which are characterized in that they consist only of the recited elements and steps.

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of and from the specified value, in particular variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

All documents cited in the present specification are hereby incorporated by reference in their entirety.

Unless otherwise specified, all terms used in disclosing the invention, including technical and scientific terms, have the meaning as commonly understood by one of ordinary skill in the art to which this invention belongs. By means of further guidance, term definitions may be included to better appreciate the teaching of the present invention.

The present invention is based on the observation that glycoprotein enriched compositions can be advantageously used to reduce the incidence and severity of enteric diseases.

While the use of blood plasma as feed additive for the prevention of diarrhoea by *E. coli* has been vastly described in literature, the exact mechanism and active ingredients on the blood plasma is difficult to identify. While it has been generally believed that the active ingredient in the blood plasma are the immunoglobulins, the inventors have now surprisingly found that glycoprotein enriched compositions where the non-immunoglobulin glycoprotein fraction is enriched provides surprisingly good results and significant positive effects on treatment and/or prevention of enteric diseases. In vivo treatment of animals with the glycoprotein enriched compositions according to the present invention showed a positive effect on the faecal score in the sub-acute phase of diarrhoea.

Whereas typical blood plasma compositions according to the prior art were specifically directed towards providing an influence on the immune system, the glycoprotein enriched compositions according to the present invention directly interact with the pathogenic organisms and not with the immune system. More particularly, the glycoprotein enriched composition according to the present invention has been found to be particularly useful and active against adherence of pathogenic organisms to the intestine. These pathogenic organisms include but are not limited to bacteria such as *E. coli, Salmonella, Clostridium, Campylobacter*, *Shigella*, etc., viruses such as norovirus, rotavirus, adenovirus, astrovirus, parvovirus, cytomegalovirus, etc. and parasites such as Giardia, Entamoeba, Cryptosporidium, etc.

Thus a first aspect of the invention relates to glycoprotein enriched compositions comprising an at least 10% higher concentration of glycoproteins compared to the natural glycoprotein source wherein the enriched glycoprotein fraction consist essentially of non intermolecular SS-bound glycoprotein-subunits as measured by non-reduced and reduced SDS-PAGE. More preferably, said glycoprotein enriched compositions comprise an at least 15%, at least 20%, at least 25%, at least 30%, at least 35%, at least 40%, at least 45%, at least 50%, at least 60%, at least 70%, at least 80%, at least 90% or at least 100% higher concentration of glycoproteins compared to the natural glycoprotein source, wherein the enriched glycoprotein fraction consist essentially of non intermolecular SS-bound glycoprotein-subunits, thereby excluding immunoglobulin fraction of the glycoproteins. Accordingly, the glycoprotein enriched composition of the present invention provides in an enriched non-immunoglobulin glycoprotein fraction compared to the non-immunoglobulin glycoprotein fraction of the natural glycoprotein source.

Whereas the term "glycoprotein enriched composition" generally relates to the composition as a whole, the enriched composition being obtained from for instance a blood plasma product, the term "enriched glycoprotein fraction" pertains specifically to a fraction of the glycoprotein enriched composition that is specifically enriched. In the compositions according to the present invention, especially the glycoprotein fraction that does not contain immunoglobulins is enriched.

The non-immunoglobulin glycoprotein fraction or glycoprotein fraction consisting essentially of non intermolecular SS-bound glycoprotein-subunits can be measured using common biochemical techniques such as for instance by performing a separation of the protein fraction according to their electrophoretic mobility on a SDS-PAGE. A comparison of the separation on an SDS-PAGE gel under non-reduced and reduced circumstances provides a measurement of the glycoprotein fraction consisting of non intermolecular SS-bound glycoprotein-subunits as the SDS-PAGE gel separation under reducing circumstances (e.g. by briefly boiling the protein fraction or performing the separation in the presence of a reducing agent, such as dithiothreitol (DTT) or 2-mercaptoethanol (betamercaptoethanol/BME) wherein the proteins are denatured by reducing disulfide linkages.

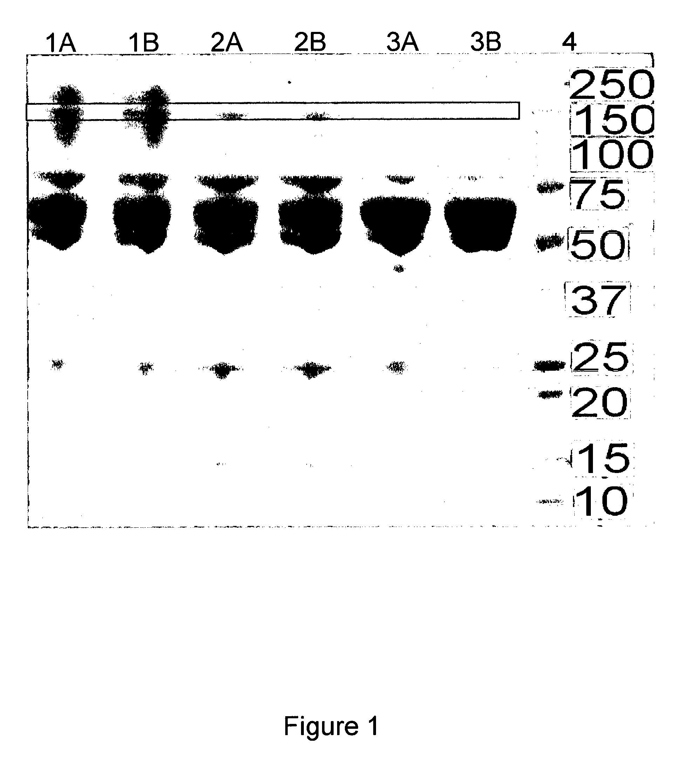

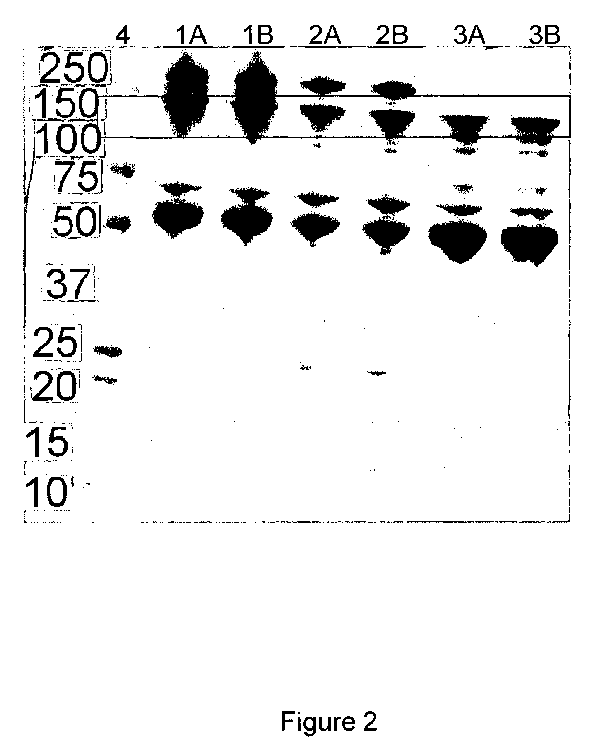

Figure 1 shows an example of such an SDS-PAGE gel separation (marker in lane 4) under reducing circumstances wherein Porcine Plasma (lanes 1A and 1B) is compared to the non-immunoglobulin glycoprotein enriched composition (lanes 2A and 2B) and the non-immunoglobulin glycoprotein depleted composition (lanes 3A and 3B). Figure 2 provides the same compositions in an SDS-PAGE gel separation under non-reducing circumstances showing the clear difference between the total glycoprotein fraction (under non-reduced conditions) and the non-immunoglobulin glycoprotein (under reduced conditions).

In a particular embodiment of the present invention, the glycoprotein enriched composition is prepared from human blood plasma, porcine blood plasma, bovine blood plasma, equine blood plasma, ovine blood plasma, avian blood plasma or any other blood plasma source typically used.

In particular embodiments said glycoprotein enriched composition represents at least 30% of the blood plasma product. More particularly, said glycoprotein enriched composition represents at least 35%, 40%, 45% or 50% of the blood plasma product.

In a further particular embodiment of the present invention, said glycoprotein enriched composition is prepared from eggs, whey or other glycoprotein containing natural sources.

In a particular embodiment of the present invention, said glycoprotein enriched composition is further characterized by comprising an at least 10% lower albumin concentration compared to the natural glycoprotein source.

In a further particular embodiment of the present invention, said glycoprotein enriched composition further comprises an immunoglobulin fraction, wherein said immunoglobulin fraction is preferably at least partially or entirely inactivated through commonly used inactivation techniques such as heat inactivation, enzymatic inactivation or any other types of commonly used inactivation techniques such as for instance but not limited to irradiation or other chemical treatments.

In a particular embodiment of the present invention, said glycoprotein enriched composition is prepared from non-immunized blood plasma. As the enriched fraction of the glycoprotein enriched composition preferably does not contain any immunoglobulin glycoproteins, it is not required that the animals from which blood plasma is used are immunized prior to obtaining the blood plasma, thereby providing a method which is completely natural and does not include immunization steps.

When used in animals or humans, the glycoprotein enriched composition of the invention can be readily administered by mixing them directly into animal feed, or separately from the feed as a supplement in an edible carrier to be later mixed with the feed.

Therefore the present invention further relates to food supplements in solid or liquid form, characterized in that said food supplement comprises a glycoprotein enriched composition according to the present invention. The inventors have additionally noted that, if administered separately from the animal feed or fodder, the glycoprotein enriched composition according to the present invention can be prepared by combining it with nontoxic acceptable edible carriers to make either immediate release or slow release formulations, as is well known in the art. Such edible carriers may be either solid or liquid such as, for example, corn starch, lactose, sucrose, soy flakes, peanut oil, olive oil, sesame oil and propylene glycol (and used as a supplement or top dressing on feed). The dosage forms may also contain adjuvants, such as preserving, stabilizing, wetting or emulsifying agents, solution promoters, etc. They may also contain other therapeutically valuable substances.

In a particular embodiment, the food supplement according to the present invention further comprises an animal feed selected from the group consisting of one or more of corn, sorghum, meat, barley, wheat, soybean meal, peanut, canola, whey, milk products, blood meal, bone meal, fish meal, fats and oils, amino acids, vitamins and minerals, to form a ration.

The glycoprotein enriched composition and food supplements according to the present invention are preferably administered orally, preferably to young animals (such as but not limited to piglets, calves, puppies, kittens, etc.) and children.

The present invention further envisages the use of the glycoprotein enriched composition according to the present invention as a feed and food additive. As used herein, the term "food" encompasses food for human consumption. As used herein, the term "feed" encompasses food for animal consumption. As used herein, the term "food or feed additive" refers to an ingredient, additive, component or supplement suitable for incorporation in human food or animal feed.

Thus, a further aspect of the invention relates to the use of the glycoprotein enriched composition according to the present invention as a food or feed additive and feed or food additives comprising the glycoprotein enriched composition according to the invention. In particular, the glycoprotein enriched composition is envisaged as a food or feed additive for subjects susceptible to gastro-intestinal disease, such as subjects after weaning. For instance, the glycoprotein enriched composition can be useful as a food or feed additive in weaned piglets.

The active agent content of a nourishment fed to the animals as a complement of the fodder, may be the 2-20 fold of the values envisaged for feed or fodder. The active agent of a premix should be increased according to its mixing ratio with the fodder or nourishment; the active agent content of a premix may be e.g. the 10-100 fold of the values envisaged for feed or fodder, more preferably a premix may be the 10, 20, 30, 40, 50, 60, 70, 80, 90 or 100 fold of the values envisaged for feed or fodder.

Examples of suitable feed materials according to the invention include but are not limited to barns, such as wheat bran, rice bran, barley bran, and millet bran; food processing byproducts, such as soybean-curd residue, starch pulp, copra meal, sake cake, soy sauce cake, brewer's grains, sweet potato distiller's residue, and juice pulp of fruits and vegetables; cereals, such as corn, rice, wheat, barley, and oat; oil seed meals, such as soybean meal, rapeseed meal, cotton seed meal, linseed meal, sesame meal, and sunflower meal; animal origin feeds, such as fish meal, casein, dried skim milk, dried whey, meat and bone meal, meat meal, feather meal, and blood meal; leaf meals, such as alfalfa meal; and the like.

In the context of the present invention, veterinary compositions are provided comprising, as an active ingredient the glycoprotein enriched composition or food or feed supplements according to the present invention.

The term "veterinary composition" encompasses the full range of compositions for internal administration and feeds and drinks which can be consumed by animals. Typical veterinary dosage forms for internal administration are orally administrable dosage forms, such as pastes, solutions, tablets, etc.. However, injectable compositions are also envisaged. The compositions of the present invention may also be medicated fodders, feeds, nutriments, premixes, drinking waters and drinking water additives. Typically, for mixing in feed, the composition is provided as a powder and for mixing in drinking water the composition is provided as a fluid.

The present invention further relates the glycoprotein enriched composition or food or feed supplements according to the present invention for use as a medicament, and more particularly, for use in the treatment and/or prevention of foodborne diseases of young mammals. The present invention further relates the use of glycoprotein enriched composition or food or feed supplements according to the present invention in the manufacture of a medicament.

In particular the present invention relates to the glycoprotein enriched composition or food or feed supplements according to the present invention for use in the treatment and/or prevention of gastro-intestinal disease and more particularly for use in the treatment and/or prevention of enteric disease, preferably for use in the treatment and/or prevention of diarrhoea, and more preferably post weaning diarrhoea. The present invention further relates the use of glycoprotein enriched composition or food or feed supplements according to the present invention in the manufacture of a medicament for the treatment and/or prevention of gastro-intestinal disease and more particularly the treatment and/or prevention of enteric disease, preferably the treatment and/or prevention of diarrhoea, and more preferably post weaning diarrhoea.

The present invention therefore also relates to methods for the treatment and/or prevention of gastro-intestinal diseases or foodborne diseases of young mammals by administering the glycoprotein enriched composition or food or feed supplements according to the present invention.

The term "gastro-intestinal disease", as used herein, refers to a disease of the digestive tract such as diseases of the esophagus, stomach, first, second and third part of the duodenum, jejunum, ileum, the ileo-cecal complex, large intestine, sigmoid colon and rectum. Gastro-intestinal diseases, in particular gastro-intestinal diseases characterized by diarrhoea, can be linked to a number of causative agents. The causative agents can be but are not limited to bacteria, viruses, stress factors, nutrition, etc. The major bacteria which cause gastro-intestinal diseases are *Escherichia coli* (*E*. *coli*) and members of the genera *Clostridium*, *Lawsonia* and *Brachyspira*. The major viruses which cause gastro-intestinal diseases are rotaviruses, coronaviruses and transmissive gastro-enteritis virus. Besides infection by bacteria and viruses, gastro-intestinal diseases can be caused by numerous changes, such as stress, which can be linked to irregular feed intake, feed structure, animal hygiene and housing conditions and inadequate feeder space in the pen. The gastro-intestinal disease can also be caused by (early) weaning. Indeed, it has been observed that in the early weaned body a number of changes take place such as morphological and functional alterations of the small intestine, changes in intestinal colonization with predominance of *E. coli* and weakening of the immune system. Non-infectious stress factors which are involved in the development of gastro-intestinal disease for instance in piglets can be but are not limited to age of piglets when they are weaned from their dam and sudden change of feed from sow milk that provides piglets with immunoglobulins.

Thus, in particular embodiments of the invention, the gastro-intestinal disease is an enteric disease. The term "enteric disease", as used herein, refers to a disease related to or associated with the intestine or bowel. The gastro-intestinal or enteric disease can be characterized by diarrhoea. The term "diarrhoea", as used herein, refers to a condition of having three or more loose or liquid stools or bowel movements per day.

The term "weaning" as used herein refers to introducing an infant to what will be its adult diet and withdrawing the supply of its mother's milk or bottled substitute. The infant is considered to be "weaned" once it no longer receives any breast milk or bottled substitute. In particular embodiments, the glycoprotein enriched composition or food or feed supplements according to the present invention is envisaged for use in the treatment and prevention of gastro-intestinal disease in weaned animals, more particularly weaned farm animals, such as weaned piglets.

The inventors found through experiments that the glycoprotein enriched composition or food or feed supplements according to the present invention showed a positive effect on the treatment of gastro-intestinal disease. In particular, the inventors found that the glycoprotein enriched composition or food or feed supplements according to the present invention showed a positive effect on the treatment of diarrhoea.

Accordingly, the glycoprotein enriched composition or food or feed supplements according to the present invention are envisaged to be useful for the treatment or prevention of gastro-intestinal disease, more particularly an enteric disease, and most particularly a gastro-intestinal disease and/or enteric disease characterized by diarrhoea, in a subject in need thereof. The glycoprotein enriched composition or food or feed supplements according to the present invention are further envisaged for use in the treatment and prevention of diarrhoea.

The present invention is envisaged to be of interest for all mammals, including humans. The term "subject" as used herein thus typically denotes animals, more particularly mammals such as humans and other primates, including non-human primates such as chimpanzees and other apes and monkey species; farm animals such as cattle, sheep, pigs, goats and horses; domestic mammals such as dogs and cats; laboratory animals including rodents such as mice, rats, guinea pigs, and the like. In particular embodiments, the subject is a farm animal such as a cow, sheep, pig, goat or horse; In further particular embodiments, the invention is of particular interest for the treatment and prevention of gastro-intestinal disease in cattle, sheep and pigs. Most particularly, the invention is envisaged to be useful in non-ruminant farm animals. In particular embodiments the subject is a pig.

The concentration of the glycoprotein enriched composition or food or feed supplements according to the present invention in a veterinary composition according to the invention depends on different factors, among others on the purpose to be attained (prevention or therapy), on the severity of the already established disease and on the type of the composition concerned.

In particular embodiments, the invention provides veterinary compositions comprising the glycoprotein enriched composition or food or feed supplements according to the present invention and an excipient such as a pharmaceutically acceptable carrier. The pharmaceutically acceptable carrier can be selected from known excipients such as, but not limited to water, carboxymethyl cellulose, propylene glycol, polyethylene glycol and oil such as for instance corn oil for solutions or sodium carbonate, whey powder for powder formulations.

In particular embodiments the compositions of the present invention comprise the glycoprotein enriched composition or food or feed supplements according to the present invention as the main or only active ingredient.

The present invention also relates to the use of the glycoprotein enriched composition or food or feed supplements according to the present invention as a dietary supplement. The present invention also relates to a method for the manufacturing of a glycoprotein enriched composition or a food supplement according to the present invention, comprising the steps of:
(a) providing a blood plasma composition or natural glycoprotein containing composition; and
(b) enriching said blood plasma or natural glycoprotein containing composition such that the concentration of glycoproteins in glycoprotein enriched composition is 10% higher compared to the blood plasma or natural glycoprotein containing composition.

The enriching of said blood plasma or natural glycoprotein containing composition can be performed using different methods including generic and non generic methods, such as, but not limited to, different types of precipitation methods, adsorption methods, chromatography methods and/or filtration separation methods.

The present invention will now be illustrated by the following, non-limiting examples.

### EXAMPLES

### Example 1: Production of glycoprotein enriched compositions

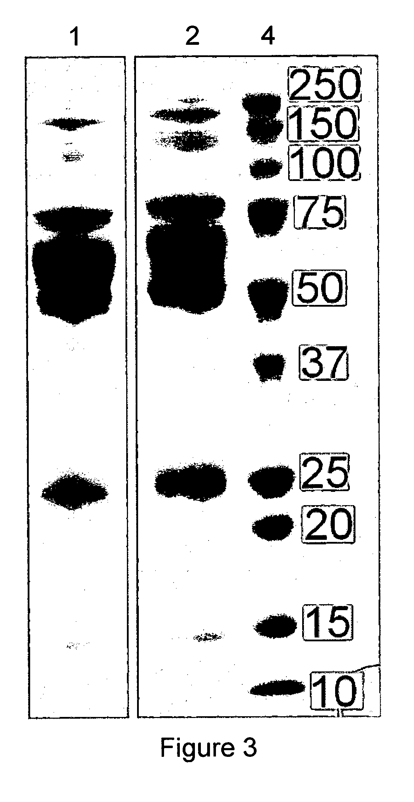

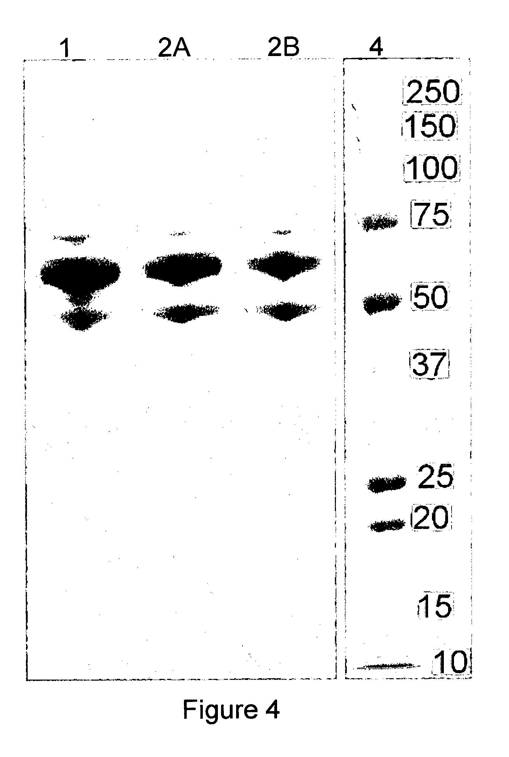

Glycoprotein enriched compositions according to particular embodiments of the present invention were prepared using chromatographic techniques. Figure 3 shows in lane number 2 the enriched composition wherein the non-immunoglobulin glycoprotein fraction was increased with 15 % compared to the non-enriched plasma fraction in lane 1. Figure 4 shows in lane number 2A and 2B the enriched composition wherein the non-immunoglobulin glycoprotein fraction was increased with respectively 26 % and 30 % compared to the non-enriched plasma fraction in lane 1.

The calculations of the degree of enrichment were made using common calculations and BioSciTec software.

### Example 2: Effects of the glycoprotein enriched compositions

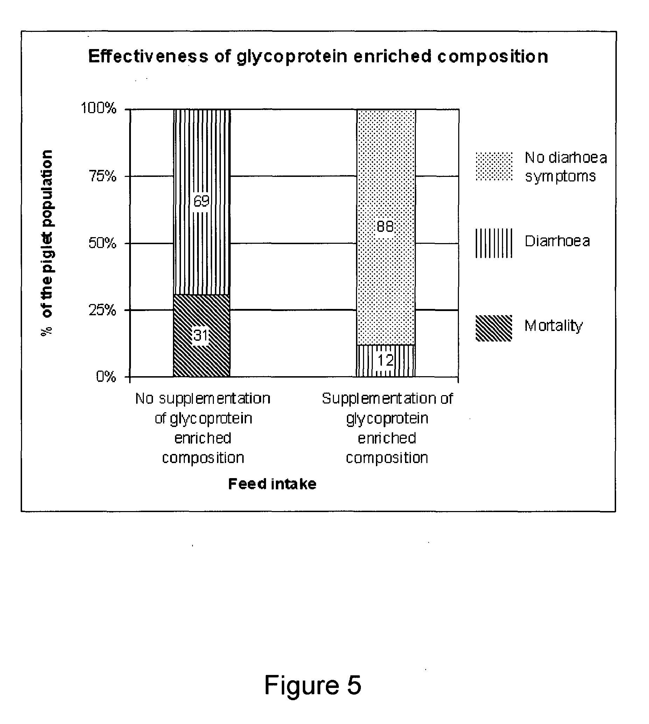

The glycoprotein enriched compositions according to the present invention were fed to early weaned pigs in an in vivo model, in which the piglets were challenged with Entero Toxigenic Escherichia coli. Figure 5 illustrates the results of these tests. Early weaned pigs which were fed with the glycoprotein enriched composition showed no mortality and a significant reduction of post weaning diarrhea.

## Claims

1. A glycoprotein enriched composition comprising an at least 10% higher concentration of glycoproteins compared to the natural glycoprotein source wherein the enriched glycoprotein fraction consist essentially of non intermolecular SS-bound glycoprotein-subunits as measured by non-reduced and reduced SDS-PAGE.

2. The glycoprotein enriched composition according to claim 1, wherein said glycoprotein enriched composition is prepared from human blood plasma, porcine blood plasma, bovine blood plasma, equine blood plasma, ovine blood plasma or avian blood plasma.

3. The glycoprotein enriched composition according to claim 1, wherein said glycoprotein enriched composition is prepared from eggs, whey or other glycoprotein containing natural sources.

4. The glycoprotein enriched composition according to any of claims 1 to 3, wherein said glycoprotein enriched composition is further **characterized by** comprising an at least 10% lower albumin concentration compared to the natural glycoprotein source.

5. The glycoprotein enriched composition according to any of claims 1 to 4, wherein said glycoprotein enriched composition is prepared from non-immunized blood plasma.

6. A food or feed supplement in solid or liquid form, **characterized in that** said food or feed supplement comprises a glycoprotein enriched composition according to any of claims 1 to 5.

7. The food or feed supplement according to claim 6, further comprising an animal feed selected from the group consisting of one or more of corn, sorghum, meat, barley, wheat, soybean meal, peanut, canola, whey, milk products, blood meal, bone meal, fish meal, fats and oils, amino acids, vitamins and minerals, to form a ration.

8. The glycoprotein enriched composition according to any of claims 1 to 5 or a food or feed supplement according to claims 6 or 7, for use as a medicament.

9. The glycoprotein enriched composition according to any of claims 1 to 5 or a food supplement according to claims 6 or 7, for use in the treatment and/or prevention of foodborne diseases of young mammals.

10. The glycoprotein enriched composition according to any of claims 1 to 5 or a food supplement according to claims 6 or 7, for use in the treatment and/or prevention of gastro-intestinal disease.

11. The glycoprotein enriched composition according to any of claims 1 to 5 or a food supplement according to claims 6 or 7, for use in the treatment and prevention of enteric disease.

12. The glycoprotein enriched composition according to any of claims 1 to 5 or a food supplement according to claims 6 or 7, for use in the treatment and prevention of diarrhoea, and more preferably post weaning diarrhoea.

13. Use of the glycoprotein enriched composition according to any of claims 1 to 5 or a food supplement according to claims 6 or 7 as a dietary supplement.

14. Method for the manufacturing of a glycoprotein enriched composition according to any of claim 1 to 5 or a food supplement according to claims 6 or 7, comprising the steps of:
(a) providing a blood plasma composition or natural glycoprotein containing composition; and
(b) enriching said blood plasma or natural glycoprotein containing composition such that the concentration of glycoproteins in glycoprotein enriched composition is 10% higher compared to the blood plasma or natural glycoprotein containing composition.

## Patentansprüche

1. Mit Glykoprotein angereicherte Zusammensetzung, die eine mindestens 10 % höhere Glykoproteinkonzentration als die natürliche Glykoproteinquelle aufweist, wobei die angereicherte Glykoproteinfraktion im Wesentlichen aus nicht-intermolekular SSgebundenen Glykoproteinuntereinheiten besteht, wie mittels nicht-reduzierender und reduzierender SDS-PAGE gemessen wird.

2. Mit Glykoprotein angereicherte Zusammensetzung nach Anspruch 1, wobei die mit Glykoprotein angereicherte Zusammensetzung aus Menschenblutplasma, Schweineblutplasma, Rinderblutplasma, Pferdeblutplasma, Schafblutplasma oder Vogelblutplasma zubereitet ist.

3. Mit Glykoprotein angereicherte Zusammensetzung nach Anspruch 1, wobei die mit Glykoprotein angereicherte Zusammensetzung aus Eiern, Molke oder anderen Glykoprotein enthaltenden natürlichen Quellen zubereitet ist.

4. Mit Glykoprotein angereicherte Zusammensetzung nach einem der Ansprüche 1 bis 3, wobei die mit Glykoprotein angereicherte Zusammensetzung ferner **dadurch gekennzeichnet ist, dass** sie eine mindestens 10 % niedrigere Albuminkonzentration als die natürliche Glykoproteinquelle aufweist.

5. Mit Glykoprotein angereicherte Zusammensetzung nach einem der Ansprüche 1 bis 4, wobei die mit Glykoprotein angereicherte Zusammensetzung aus nicht-immunisiertem Blutplasma zubereitet ist.

6. Nahrungs- oder Futtermittelergänzung in fester oder flüssiger Form, **dadurch gekennzeichnet, dass** die Nahrungs- oder Futtermittelergänzung eine mit Glykoprotein angereicherte Zusammensetzung nach einem der Ansprüche 1 bis 5 aufweist.

7. Nahrungs- oder Futtermittelergänzungsmittel nach Anspruch 6, das ferner ein Tierfuttermittel aufweist, welches aus der Gruppe ausgewählt ist, die aus mindestens einem aus Mais, Hirse, Fleisch, Gerste, Weizen, Sojabohnenmehl, Erdnuss, Kanola, Molke, Milcherzeugnissen, Blutmehl, Knochenmehl, Fischmehl, Fetten und Ölen, Aminosäuren, Vitaminen und Mineralstoffen besteht, um eine Ration zu bilden.

8. Mit Glykoprotein angereicherte Zusammensetzung nach einem der Ansprüche 1 bis 5 oder ein Nahrungs- oder Futtermittelergänzungsmittel nach Anspruch 6 oder 7 für die Verwendung als Medikament.

9. Mit Glykoprotein angereicherte Zusammensetzung nach einem der Ansprüche 1 bis 5 oder ein Nahrungsergänzungsmittel nach Anspruch 6 oder 7 für die Verwendung bei der Behandlung und/oder Prävention von über Nahrungsmittel übertragene Krankheiten junger Säugetiere.

10. Mit Glykoprotein angereicherte Zusammensetzung nach einem der Ansprüche 1 bis 5 oder ein Nahrungsergänzungsmittel nach Anspruch 6 oder 7 für die Verwendung bei der Behandlung und/oder Prävention einer Magendarmkrankheit.

11. Mit Glykoprotein angereicherte Zusammensetzung nach einem der Ansprüche 1 bis 5 oder ein Nahrungsergänzungsmittel nach Anspruch 6 oder 7 für die Verwendung bei der Behandlung und Prävention einer Darmkrankheit.

12. Mit Glykoprotein angereicherte Zusammensetzung nach einem der Ansprüche 1 bis 5 oder ein Nahrungsergänzungsmittel nach Anspruch 6 oder 7 für die Verwendung bei der Behandlung und Prävention von Durchfall und insbesondere von Durchfall nach dem Entwöhnen/Absetzen.

13. Verwendung der mit Glykoprotein angereicherten Zusammensetzung nach einem der Ansprüche 1 bis 5 oder eines Nahrungsergänzungsmittels nach Anspruch 6 oder 7 als diätetisches Ergänzungsmittel.

14. Verfahren für die Herstellung einer mit Glykoprotein angereicherten Zusammensetzung nach einem der Ansprüche 1 bis 5 oder eines Nahrungsergänzungsmittels nach Anspruch 6 oder 7, welches die folgenden Schritte umfasst:
(a) Bereitstellen einer Blutplasmazusammensetzung oder einer natürliches Glykoprotein enthaltenden Zusammensetzung; und
(b) Anreichern des Blutplasmas oder der natürliches Glykoprotein enthaltenden Zusammensetzung derart, dass die Glykoproteinkonzentration in einer mit Glykoprotein angereicherten Zusammensetzung 10 % höher ist als in dem Blutplasma oder der natürliches Glykoprotein enthaltenden Zusammensetzung.

## Revendications

1. Composition enrichie en glycoprotéines comprenant une concentration au moins 10 % supérieure en glycoprotéines comparé à la source naturelle de glycoprotéines, dans laquelle la fraction enrichie en glycoprotéines est essentiellement constituée de sous-unités de glycoprotéines à liaisons SS non intermoléculaires tel que mesuré par SDS-PAGE en conditions réductrices et non réductrices.

2. Composition enrichie en glycoprotéines selon la revendication 1, dans laquelle ladite composition enrichie en glycoprotéines est préparée à partir de plasma sanguin humain, de plasma sanguin porcin, de plasma sanguin bovin, de plasma sanguin équin, de plasma sanguin ovin ou de plasma sanguin aviaire.

3. Composition enrichie en glycoprotéines selon la revendication 1, dans laquelle ladite composition enrichie en glycoprotéines est préparée à partir d'oeufs, de lactosérum ou d'autres sources naturelles contenant des glycoprotéines.

4. Composition enrichie en glycoprotéines selon l'une quelconque des revendications 1 à 3, dans laquelle ladite composition enrichie en glycoprotéines est en outre **caractérisée en ce qu'**elle comprend une concentration en albumine au moins 10 % inférieure comparé à la source naturelle de glycoprotéines.

5. Composition enrichie en glycoprotéines selon l'une quelconque des revendications 1 à 4, dans laquelle ladite composition enrichie en glycoprotéines est préparée à partir de plasma sanguin non immunisé.

6. Aliment ou supplément alimentaire sous forme solide ou liquide, **caractérisé en ce que** ledit aliment ou supplément alimentaire comprend une composition enrichie en glycoprotéines selon l'une quelconque des revendications 1 à 5.

7. Aliment ou supplément alimentaire selon la revendication 6, comprenant en outre un aliment animal sélectionné dans le groupe constitué d'au moins l'un du maïs, du sorgo, de la viande, de l'orge, du blé, de la farine de soja, des arachides, du colza canola, du lactosérum, des produits laitiers, de la farine de sang, de la farine d'os, de la farine de poisson, des graisses et des huiles, des acides aminés, des vitamines et des minéraux, pour former une ration.

8. Composition enrichie en glycoprotéines selon l'une quelconque des revendications 1 à 5, ou aliment ou supplément alimentaire selon les revendications 6 ou 7, pour son utilisation en tant que médicament.

9. Composition enrichie en glycoprotéines selon l'une quelconque des revendications 1 à 5, ou supplément alimentaire selon les revendications 6 ou 7, pour son utilisation dans le traitement et/ou la prévention de maladies d'origine alimentaire chez les jeunes mammifères.

10. Composition enrichie en glycoprotéines selon l'une quelconque des revendications 1 à 5, ou supplément alimentaire selon les revendications 6 ou 7, pour son utilisation dans le traitement et/ou la prévention d'une maladie gastro-intestinale.

11. Composition enrichie en glycoprotéines selon l'une quelconque des revendications 1 à 5, ou supplément alimentaire selon les revendications 6 ou 7, pour son utilisation dans le traitement et/ou la prévention d'une maladie entérique.

12. Composition enrichie en glycoprotéines selon l'une quelconque des revendications 1 à 5, ou supplément alimentaire selon les revendications 6 ou 7, pour son utilisation dans le traitement et la prévention de la diarrhée, et de manière d'avantage préférée de la diarrhée post-sevrage.

13. Utilisation de la composition enrichie en glycoprotéines selon l'une quelconque des revendications 1 à 5, ou d'un supplément alimentaire selon les revendications 6 ou 7 en tant que supplément alimentaire.

14. Procédé de fabrication d'une composition enrichie en glycoprotéines selon l'une quelconque des revendications 1 à 5, ou d'un supplément alimentaire selon les revendications 6 ou 7, ledit procédé comprenant les étapes de :
(a) fourniture d'une composition de plasma sanguin ou d'une composition contenant une glycoprotéine naturelle ; et
(b) enrichissement de ladite composition contenant du plasma sanguin ou des glycoprotéines naturelles de telle sorte que la concentration en glycoprotéines dans la composition enrichie en glycoprotéines est 10 % supérieure comparé à la composition contenant du plasma sanguin ou des glycoprotéines naturelles.
